# FASCICULE DE BREVET EUROPEEN

(11) **EP 2 283 873 B1**
(45) Date de publication et mention de la délivrance du brevet: **21.03.2012**
(21) Numéro de dépôt: 10305766.7
(22) Date de dépôt: 09.07.2010
(51) Int. Cl.: A61L 2/20, A61L 2/24

(54) **Installation de stérilisation d'une pluralité d'objets et procédé de stérilisation associé**
Sterilisierungsanlage für eine Vielzahl von Gegenständen und entsprechendes Sterilisierungsverfahren
Sterilisation installation for a plurality of objects and associated sterilisation method

(30) Priorité: 10.07.2009 FR 0954838
(43) Date de publication de la demande: 16.02.2011
(73) Titulaire: PE Investissement, 60173 Ivry-le-Temple (FR)
(72) Inventeur: Perouse, Eric, 75016, PARIS (FR)
(74) Mandataire: Domenego, Bertrand

(56) Documents cités:
- EP-A2- 0 624 518
- WO-A1-2007/000639
- GB-A- 2 326 817
- US-A1- 2002 119 074

## Description

La présente invention concerne une installation de stérilisation d'une pluralité d'objets, du type comprenant :
- un poste d'entrée, propre à recevoir la pluralité d'objets à stériliser;
- un poste de stérilisation comprenant au moins une enceinte de stérilisation destinée à recevoir la pluralité d'objets, le poste de stérilisation comprenant au moins un moyen d'introduction d'un fluide de stérilisation dans l'enceinte de stérilisation et au moins un moyen de purge du fluide de stérilisation ;
- un poste de désorption comprenant au moins une enceinte de désorption, le poste de désorption comportant au moins un moyen de purge de l'enceinte de désorption ;
- un poste de sortie propre à recevoir la pluralité d'objets après stérilisation ;
- des moyens de transport de la pluralité d'objets, propres à déplacer successivement la pluralité d'objets depuis le poste d'entrée, dans le poste de stérilisation et dans le poste de désorption, jusqu'au poste de déchargement.

Une telle installation s'applique à la stérilisation de dispositifs médicaux destinés à entrer en contact avec une surface corporelle d'un patient. Ces dispositifs médicaux sont par exemple des outils médicaux, chirurgicaux, ou dentaires, des seringues d'injection ou de prélèvement, des cathéters, ou des implants tels que des prothèses vasculaires, des chambres implantables, et plus généralement des dispositifs médicaux en contact avec le corps humain ou animal.

L'installation est conçue pour stériliser simultanément un grand nombre de dispositifs médicaux, afin de répondre aux normes applicables pour une utilisation fiable et sûre de ces dispositifs.

Une installation connue du type précité comprend généralement un poste d'entrée dans lequel des palettes de dispositifs médicaux à stériliser sont déchargés.

L'installation comprend en outre un poste de pré-conditionnement dans lequel les dispositifs médicaux contenus dans les palettes sont soumis à une atmosphère contrôlée en hygrométrie et en température pendant une durée allant de huit à douze heures.

L'installation comporte ensuite un poste de stérilisation muni d'une enceinte de stérilisation, généralement formée par un autoclave de grand volume.

Dans cette enceinte, les dispositifs médicaux sont placés en contact avec un gaz stérilisant tel que l'oxyde d'éthylène, pendant une durée par exemple égale à huit heures.

L'installation comprend de plus un poste de désorption muni d'enceintes de désorption où les dispositifs médicaux subissent, pendant une durée de seize à trente-deux heures, une désorption pour extraire l'oxyde d'éthylène résiduel.

Une telle installation est donc très étendue, notamment lorsque le nombre de dispositifs médicaux à traiter est important. En effet, le temps de traitement de chaque lot de dispositifs est élevé, ce qui requiert un espace important pour stocker les lots entre deux postes.

En outre, pour passer d'un poste à l'autre, les dispositifs médicaux contenus dans des palettes sont transportés par des véhicules à fourches depuis le poste d'entrée jusqu'au poste de pré-conditionnement, du poste de pré-conditionnement au poste de stérilisation, du poste de stérilisation au poste de désorption, et enfin du poste de désorption au poste de sortie.

Une telle installation nécessite donc, lorsque le nombre de palettes à traiter est important, un grand nombre de manipulations, ce qui est coûteux et nécessite des moyens humains importants.

En outre, de grandes précautions doivent être prises pour empêcher que le personnel ne soit exposé aux gaz stérilisants qui sont parfois irritants, voire nocifs.

Enfin, ce type d'installation ne garantit pas que des dispositifs médicaux soient transportés depuis l'étape de pré-conditionnement vers le poste de désorption sans passer par le poste de stérilisation, ce qui peut être potentiellement dangereux. Pour pallier ce risque, il est nécessaire de construire des cloisons étanches entre tous les postes, ce qui est coûteux et encombrant.

Une autre installation de stérilisation d'object est connue par le document US-A-2002/0119074.

Un but de l'invention est donc d'obtenir une installation de stérilisation capable de traiter un grand nombre d'objets à stériliser de manière peu coûteuse, très sûre pour le personnel, et très fiable.

A cet effet, l'invention a pour objet une installation de stérilisation du type précité, caractérisé en ce que les moyens de transport comprennent :
- une voie de guidage continue, le poste d'entrée, le poste de stérilisation, le poste de désorption et le poste de déchargement étant disposés successivement en regard de la voie de guidage ; et
- au moins un engin guidé par la voie de guidage, l'engin guidé comprenant des moyens de déplacement propres à déplacer la pluralité d'objets par rapport à l'engin guidé entre une position de déplacement conjoint de la pluralité d'objets et de l'engin guidé le long de la voie de guidage, et une position d'insertion de la pluralité d'objets dans chacune de l'enceinte de stérilisation et de l'enceinte de désorption.

L'installation selon l'invention peut comprendre une ou plusieurs des caractéristiques suivantes, prise(s) isolément ou suivant toute(s) combinaison(s) techniquement possible(s) :
- elle comporte au moins un conteneur délimitant un volume de réception de la pluralité d'objets, le conteneur comprenant au moins un organe de raccordement libérable aux moyens de déplacement ;
- la voie de guidage est située au-dessus de l'enceinte de stérilisation et au-dessus de l'enceinte de désorption, l'enceinte de stérilisation et l'enceinte de désorption débouchant vers le haut en regard de la voie de guidage ;
- l'engin guidé comprend un pont roulant, les moyens de déplacement comprenant au moins un organe de traction porté par le pont roulant et propre à descendre la pluralité d'objets dans l'enceinte de stérilisation et dans l'enceinte de désorption et à remonter la pluralité d'objets pour les extraire hors de l'enceinte de stérilisation et de l'enceinte de désorption ;
- l'enceinte de stérilisation et l'enceinte de désorption délimitent chacune une ouverture unique d'accès pour l'insertion et le retrait de la pluralité d'objets ;
- la voie de guidage s'étend linéairement entre le poste d'entrée et le poste de sortie, le poste de stérilisation et le poste de désorption étant situés côte à côte le long de la voie de guidage entre le poste d'entrée et le poste de sortie ;
- le poste de stérilisation comprend une pluralité d'enceintes de stérilisation, le poste de désorption comprenant une pluralité d'enceintes de désorption, les enceintes de stérilisation et les enceintes de désorption étant disposées successivement côte à côte de manière adjacente le long de la voie de guidage ;
- elle comprend un poste de préconditionnement disposé en amont du poste de stérilisation le long de la voie de guidage, le poste de préconditionnement comprenant au moins une enceinte de préconditionnement et des moyens de contrôle de l'humidité et/ou de la température dans l'enceinte de préconditionnement ;
- elle comprend une paroi de confinement délimitant un volume de confinement, le poste de stérilisation et le poste de désorption étant situés dans le volume de confinement, le poste d'entrée et le poste de sortie étant situés hors du volume de confinement,
et la paroi de confinement délimite un sas amont et un sas aval débouchant dans le volume de confinement, la voie de guidage traversant successivement le sas amont, le volume de confinement et le sas aval ;
- le poste de sortie comprend au moins un véhicule roulant délimitant une surface de chargement de la pluralité d'objets stérilisés, le véhicule roulant étant mobile entre une position de réception de la pluralité d'objets dans laquelle la surface de chargement s'étend en regard de la voie de guidage pour être chargée par les moyens de transport et une position à l'écart de la voie de guidage ;
- l'enceinte de stérilisation délimite une chambre de stérilisation propre à recevoir la pluralité d'objets et comprend un ouvrant d'obturation étanche de la chambre de stérilisation, le poste de stérilisation comprenant des moyens de déplacement de l'ouvrant propre à déplacer l'ouvrant entre une position d'accès à la chambre de stérilisation et une position d'obturation de la chambre de stérilisation ;
- dans la position d'accès, la chambre de stérilisation débouche en regard de la voie de guidage, l'ouvrant étant décalé transversalement par rapport à la voie de guidage ; et
- les moyens de déplacement de l'ouvrant comprennent un engin auxiliaire guidé par la voie de guidage.

L'invention a en outre pour objet un procédé stérilisation d'une pluralité d'objets dans une installation telle que définie ci-dessus, caractérisé en ce qu'il comprend les étapes suivantes :
- saisie de la pluralité d'objets dans le poste d'entrée par les moyens de déplacement portés par l'engin guidé,
- déplacement de l'engin guidé sur la voie de guidage pour le placer en regard du poste de stérilisation ;
- introduction de la pluralité d'objets dans l'enceinte de stérilisation par les moyens de déplacement portés par l'engin guidé ;
- injection de fluide stérilisant dans l'enceinte de stérilisation pour stériliser la pluralité d'objets dans l'enceinte de stérilisation ;
- extraction de la pluralité d'objets hors de l'enceinte de stérilisation par les moyens de déplacement portés par l'engin guidé ;
- déplacement de l'engin guidé sur la voie de guidage pour le placer en regard du poste de désorption ;
- introduction de la pluralité d'objets dans l'enceinte de désorption par les moyens de déplacement portés par l'engin guidé ;
- désorption du fluide de stérilisation hors de la pluralité d'objets dans l'enceinte de désorption ;
- extraction de la pluralité d'objets hors de l'enceinte de désorption par les moyens de déplacement portés par l'engin guidé ;
- déplacement de l'engin guidé sur la voie de guidage jusqu'au poste de sortie ;
- dépôt de la pluralité d'objets stérilisés dans le poste de sortie par les moyens de déplacement portés par l'engin guidé.

L'invention sera mieux comprise à la lecture de la description qui va suivre, donnée uniquement à titre d'exemple et faite en se référant aux dessins annexés, sur lesquels :
- la Figure 1 est une vue schématique de dessus d'une première installation selon l'invention ;
- la Figure 2 est une vue partielle de côté d'un poste de l'installation de la Figure 1, lors du chargement dans le poste d'un conteneur recevant une pluralité d'objets ;
- la Figure 3 est une vue analogue à la Figure 2, lors du traitement de la pluralité d'objets dans le poste ;
- la Figure 4 est une vue de dessus des parties pertinentes du poste représenté sur la Figure 2 ;
- la Figure 5 est une vue de côté d'un conteneur destiné à recevoir une pluralité de palettes transportées à travers l'installation de stérilisation selon l'invention ;
- la Figure 6 est une vue analogue à la Figure 5 d'une variante de conteneur ;
- la Figure 7 est une vue partielle de dessus de l'engin guidé d'une deuxième installation selon l'invention.

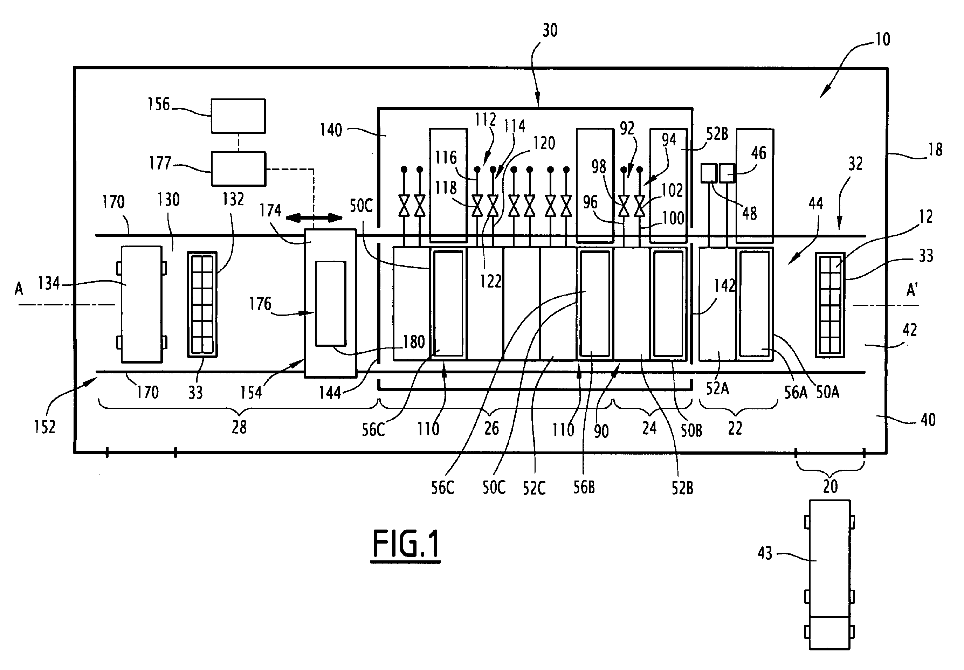

Une première installation 10 de stérilisation selon l'invention est représentée sur les Figures 1 à 4.

Cette installation 10 est destinée à stériliser une pluralité d'objets formés avantageusement par des dispositifs médicaux propres à entrer en contact avec une surface corporelle d'un patient.

Ces dispositifs médicaux sont par exemple, des outils médicaux, chirurgicaux, ou dentaires, des seringues d'injection ou de prélèvement, des cathéters, ou des implants tels que des prothèses vasculaires, des chambres implantables.

Ces objets sont avantageusement contenus dans des boîtes, les boîtes étant elles-mêmes stockées dans des palettes 12, visibles sur les Figures 1 à 5. Chaque palette 12 contient généralement plus d'une dizaine d'objet, voire plus d'une centaine d'objets.

Dans l'exemple illustré sur la Figure 1, la première installation 10 de stérilisation est logée dans un bâtiment unique 18.

Elle comprend, d'amont en aval le long d'un axe horizontal A-A' de déplacement des objets, un poste 20 d'entrée, pour la réception des palettes 12 contenant des objets à stériliser, un poste 22 de pré-conditionnement, un poste 24 de stérilisation, et un poste 26 de désorption.

Elle comprend en outre un poste 28 de sortie, pour recevoir les palettes 12 d'objets stérilisés et les expédier vers un lieu de stockage ou d'utilisation.

L'installation 10 comprend en outre une paroi 30 de confinement du poste de stérilisation 24 et du poste de désorption 26, et des moyens 32 de transport des palettes 12 d'objets à travers successivement les postes 20 à 28, les moyens de transport 32 comprenant au moins un conteneur 33 de manipulation simultanée d'une pluralité de palettes 12.

Le poste d'entrée 20 comprend un quai de déchargement 40 pour recevoir les palettes 12 d'objets à stériliser provenant de l'extérieur du bâtiment 18 et un plateau d'entrée 42, sur lequel les palettes 12 sont placées dans un conteneur 33 en vue de leur prise en charge par les moyens de transport 32.

Dans cet exemple, le quai 40 est adapté pour recevoir au moins un camion 43 qui contient des palettes 12 d'objets non stériles. Le plateau 42 comprend une surface horizontale d'appui des palettes 12 et du conteneur 33.

Le poste de pré-conditionnement 22 comprend au moins une enceinte 44 de pré-conditionnement, des moyens 46 de réglage de la température dans l'enceinte 44, et des moyens 48 de réglage de l'hygrométrie dans l'enceinte 44.

Dans l'exemple représenté sur la Figure 1, le poste de pré-conditionnement 22 comprend deux enceintes 44 identiques, situées côte à côte de manière adjacente. Les enceintes 44 s'étendent sensiblement perpendiculairement à l'axe de déplacement A-A'.

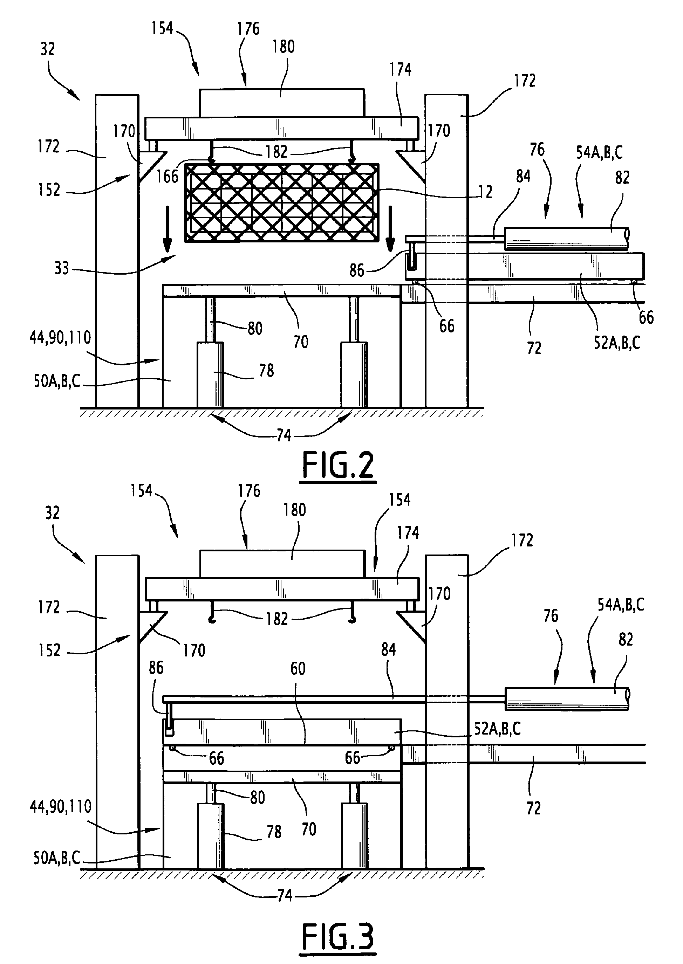

Comme illustré par la Figure 2, chaque enceinte 44 comprend une cuve de pré-conditionnement 50A, un ouvrant formant couvercle amovible 52A et des moyens 54 de déplacement du couvercle amovible 52A entre une position d'obturation de la cuve 50A et une position d'accès à la cuve 50A.

La cuve 50A est de forme sensiblement parallélépipédique allongée suivant un axe perpendiculaire à l'axe A-A'. Elle délimite une chambre de pré-conditionnement 56A débouchant vers le haut par une ouverture supérieure 58.

L'ouverture supérieure 58 est délimitée à sa périphérie par un rebord supérieur 60 de la cuve 50A. Le rebord 60 reçoit le couvercle 52A en appui dans sa position d'obturation.

Le rebord 60 porte un joint 62 d'étanchéité s'étendant à la périphérie de l'ouverture supérieure 58 pour assurer l'étanchéité à travers l'ouverture supérieure 58 lorsque le couvercle 52A obture l'ouverture supérieure 58.

La chambre 56A présente une forme sensiblement parallélépipédique analogue à celle du conteneur 33. Le volume de la chambre de pré-conditionnement 56A est légèrement supérieur, par exemple au plus 20% supérieur, au volume du conteneur 33 destiné à recevoir les palettes 12.

Ce volume est par exemple supérieur à 1m³ et est compris avantageusement entre 1m³ et 100m³.

Le couvercle 52A comprend un panneau d'obturation 64, de largeur légèrement supérieure à la largeur de la cuve 50A. Il comporte des organes de roulage 66 montés le long des bords latéraux du panneau 64 sur une surface inférieure du panneau 64.

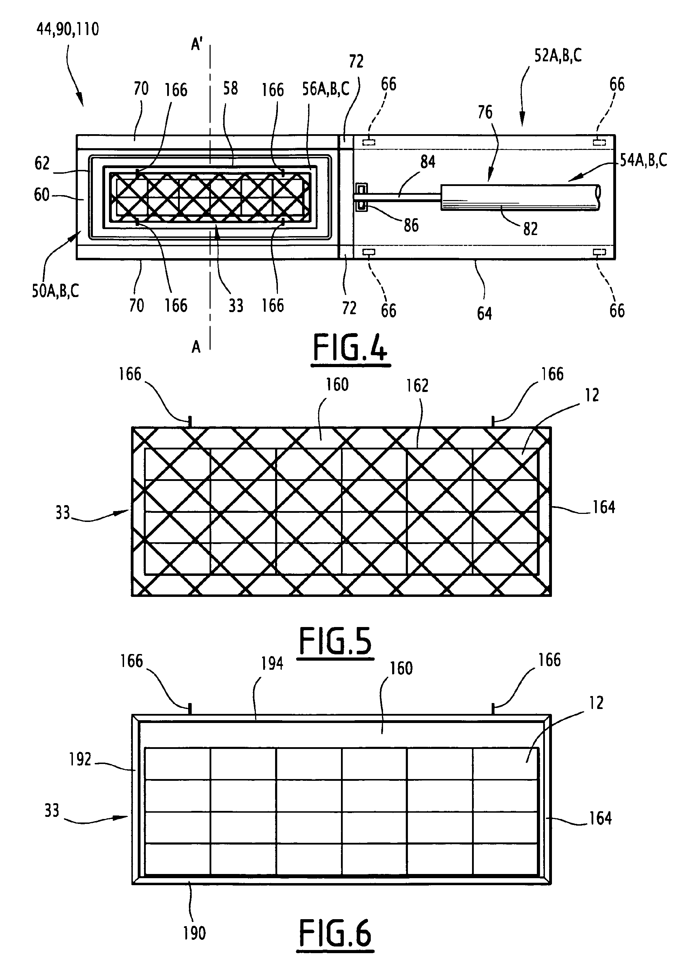

Le couvercle 52A est déplaçable transversalement par rapport à l'axe A-A' entre une position d'accès à la chambre 56A, représentée sur les Figures 2 et 4, et une position d'obturation représentée sur la Figure 3.

Dans la position d'accès, le panneau 64 a été décalé transversalement à l'écart de l'ouverture supérieure 58. Le volume situé au-dessus de l'ouverture supérieure 58 est dégagé sur au moins la hauteur d'un conteneur 33 pour permettre l'introduction par le haut du conteneur 33 contenant les palettes 12 dans la chambre de pré-conditionnement 56A.

Dans la position d'obturation, le panneau 64 est appliqué contre le rebord supérieur 60. A l'aide du joint 62, le panneau 64 obture de manière étanche vers le haut l'ouverture supérieure 58 et empêche l'accès depuis l'extérieur à la chambre de pré-conditionnement 56A.

Dans l'exemple représenté sur la Figure 2, les moyens de déplacement 54A comprennent des rails mobiles 70 disposés latéralement le long de la cuve 50A, des rails fixes 72 décalés transversalement par rapport à la cuve 50A, des premiers vérins 74 de déplacement vertical des rails mobiles 70 et au moins un deuxième vérin 76 de déplacement horizontal du couvercle 52A sur les rails 70, 72.

Les rails mobiles 70 s'étendent transversalement par rapport à l'axe A-A', de part et d'autre de la cuve 50A. Ils sont portés par les premiers vérins 74.

Chaque rail 70 est déplaçable sous l'effet des premiers vérins 74 entre une position inférieure de repos, représentée sur la Figure 3 et une position supérieure de roulage du couvercle, représentée sur la Figure 2.

Dans la position inférieure, les rails mobiles 70 sont situés au dessous des rails fixes 72, et au dessous du rebord 60.

Dans la position supérieure, chaque rail mobile 70 est situé sensiblement au droit du rebord 60, en regard du rail fixe 72. La surface supérieure de chaque rail mobile 70 affleure alors la surface supérieure d'un rail fixe 72, ces surfaces supérieures définissant une surface horizontale continue de guidage des organes de roulage 66. La surface de guidage s'étend transversalement par rapport à l'axe A-A'.

Chaque rail fixe 72 s'étend verticalement au droit du rebord 60, en dessous du deuxième vérin 76. Le rail fixe 72 s'étend en outre à l'écart transversalement de la cuve 50A.

Chaque premier vérin 74 comprend un cylindre 78 et une tige 80 verticale portant le rail mobile 70.

Dans l'exemple représenté sur la Figure 2, les moyens de déplacement 54 comprennent, pour chaque rail mobile 70, deux premiers vérins parallèles 74 portant deux points espacés transversalement du rail mobile 70.

Le deuxième vérin 76 comprend un cylindre horizontal 82 disposé au-dessus du couvercle 52A et une tige horizontale 84 déployable à partir du cylindre horizontal 82. La tige horizontale 84 présente une extrémité libre 86 fixée sur le couvercle 52A le long d'un axe transversal de déplacement de la tige 84, tout en autorisant un déplacement vertical relatif du couvercle 52A par rapport à la tige 84.

Ainsi, la tige horizontale 84 est mobile entre une configuration rétractée dans le cylindre 82, dans laquelle le couvercle 52A occupe sa position d'accès et une configuration déployée, dans laquelle le couvercle 52A a été déplacé sur les rails 72, 70 en regard de l'ouverture supérieure 58 pour obturer cette ouverture 58.

Les moyens 46 de réglage de la température sont aptes à réguler la température dans la chambre de pré-conditionnement 56A pour que cette température soit comprise à une température donnée choisie comprise par exemple entre 30°C et 60°C.

Les moyens 48 de réglage de l'hygrométrie sont aptes à régler le pourcentage d'humidité dans la chambre de pré-conditionnement 56A à une valeur donnée comprise entre 40% et 100% d'humidité relative.

Le poste de stérilisation 24 comprend au moins une enceinte 90 de stérilisation et, pour chaque enceinte 90, des moyens 92 d'introduction d'un gaz stérilisant dans l'enceinte 90 et des moyens 94 de purge de l'enceinte 90.

Dans l'exemple représenté sur la Figure 1, le poste de stérilisation 24 comprend deux enceintes 90 identiques.

Les enceintes 90 s'étendent transversalement par rapport à l'axe A-A' de déplacement. Elles sont placées côte à côte de manière adjacente le long de l'axe A-A'.

Au moins une enceinte de stérilisation 90 s'étend de manière adjacente à une enceinte de pré-conditionnement 44 du poste de pré-conditionnement 22 pour minimiser l'espace occupé par l'installation 10 dans le bâtiment 18.

L'enceinte de stérilisation 90 présente une structure analogue à celle de l'enceinte de pré-conditionnement 44. Ainsi, en référence aux Figures 1 à 3, l'enceinte 90 comprend une cuve de stérilisation 50B, un couvercle 52B et des moyens 54B de déplacement du couvercle 52B entre une position d'obturation de la cuve 50B et une position d'accès à la cuve 50B.

La cuve 50B délimite intérieurement une chambre de stérilisation 56B qui débouche vers le haut.

La cuve 50B, le couvercle 52B et les moyens de déplacement 54B sont de structures analogues respectivement à celle de la cuve de pré-conditionnement 50A, à celle du couvercle 52A et à celle des moyens de déplacement 54A décrits plus haut et ne seront donc pas décrits plus en détail.

Comme illustré par la Figure 1, les moyens 92 d'introduction d'un gaz stérilisant comprennent une conduite 96 d'introduction de gaz stérilisant, la conduite 96 est munie d'une vanne 98 de commande.

Cette conduite 96 raccorde un réservoir (non représenté) de gaz stérilisant à la chambre de stérilisation 56B. Le gaz stérilisant est avantageusement de l'oxyde d'éthylène.

Les moyens de purge 94 comprennent une conduite de purge 100 munie d'une vanne de purge 102. La conduite de purge 100 débouche dans la chambre 56B de stérilisation. Elle est raccordée en aval à une pompe à vide.

Le poste de désorption 26 comprend au moins une enceinte de désorption 110, des moyens 112 d'introduction d'un gaz de rinçage dans l'enceinte de désorption 110, et des moyens 114 de purge des gaz désorbés.

Dans l'exemple représenté sur la Figure 1, le poste 26 comporte au moins deux fois plus, avantageusement au moins trois fois plus d'enceintes de désorption 110 que le poste de stérilisation 24 ne comprend d'enceintes 90 de stérilisation. Ainsi, le nombre d'enceintes 110 représentées sur la Figure 1 est égal à six.

Les enceintes 110 s'étendent transversalement par rapport à l'axe A-A' de déplacement. Elles sont placées côte à côte de manière adjacente le long de l'axe A-A'.

Une enceinte de désorption 110 s'étend de manière adjacente à une enceinte de stérilisation 90 du poste de stérilisation 24 pour minimiser l'espace occupé par l'installation 10 dans le bâtiment 18.

Chaque enceinte de désorption 110 présente une structure analogue à celle de l'enceinte de stérilisation 90 et de l'enceinte de pré-conditionnement 44.

Ainsi, en référence aux Figures 1 à 3, chaque enceinte 110 comprend une cuve 50C de désorption, un couvercle 52C, et des moyens 54C de déplacement du couvercle 52C entre une position d'obturation de la cuve 50C et une position d'accès à la cuve 50C.

La cuve 50C délimite intérieurement une chambre de désorption 56C qui débouche vers le haut. La cuve 50C, le couvercle 52C et les moyens de déplacement 54C sont de structures analogues respectivement à celle de la cuve de pré-conditionnement 50A, à celle du couvercle 52A et à celle des moyens de déplacement 54A décrits plus haut et ne seront donc pas décrits plus en détail.

Les moyens 112 d'introduction d'un gaz de rinçage comprennent une conduite d'introduction 116 raccordée à un réservoir (non représenté) de gaz de rinçage. La conduite d'introduction 116 est munie d'une vanne de contrôle 118. Le gaz de rinçage est par exemple un gaz neutre, tel que l'azote.

Les moyens de purge 114 comprennent une conduite de purge 120 munie d'une vanne de contrôle 122, la conduite 120 étant raccordée à une pompe à vide (non représentée).

Le poste de sortie 28 comprend un plateau de sortie 130 présentant une région de stockage 132 des palettes 12 d'objets stérilisés.

Dans l'exemple représenté sur la Figure 1, le poste de sortie 28 comprend en outre au moins un véhicule roulant 134 destiné à recevoir les palettes 12 d'objets stérilisés en vue de leur transport ultérieur hors du bâtiment 18.

Le véhicule roulant 134 est par exemple une remorque de camion. Le véhicule 134 est ainsi déplaçable entre une position de réception des palettes 12 d'objets dans laquelle il s'étend en regard des moyens de transport 32 et une position à l'écart des moyens de transport 32, située hors du bâtiment 18.

La paroi de confinement 30 s'étend autour du poste de stérilisation 24 et du poste de désorption 26. Elle entoure ces postes 24, 26 à leur périphérie et les recouvre.

La paroi 30 délimite un volume intérieur 140 de confinement, d'atmosphère contrôlée, dans laquelle sont disposées les enceintes de stérilisation 90 et les enceintes de désorption 110.

La longueur du volume de confinement 140, prise le long de l'axe A-A', est par ailleurs sensiblement égale à la longueur cumulée occupée par les cuves de stérilisation 50B et par les cuves de désorption 50C le long de l'axe A-A'.

La paroi de confinement 30 délimite un sas amont 142 et un sas aval 144 pour le passage des moyens de transport 32.

Le sas amont 142 débouche au-dessus des enceintes de stérilisation 90 entre le poste de pré-conditionnement 22 et le poste de stérilisation 24. Il est centré sur l'axe de déplacement A-A'.

Le sas aval 144 débouche en regard du sas amont 142 le long de l'axe A-A'. Il débouche entre le poste de pré-conditionnement 26 et le poste de sortie 28 au dessus des enceintes de désorption 110.

Selon l'invention, les moyens de transport 32 comprennent, outre le conteneur 33 de transport des palettes 12, une voie de guidage 152 s'étendant linéairement le long de l'axe A-A' le long des différents postes 20 à 28, et un engin guidé 154 de déplacement du conteneur 33 entre les différents postes 20, 28. L'engin 154 est monté roulant sur la voie de guidage 152.

Les moyens de transport 32 comprennent en outre des moyens 156 de commande du déplacement de l'engin guidé 154.

Le conteneur 33 est de forme sensiblement analogue à la forme de la chambre de pré-conditionnement 56A, à la forme de la chambre de stérilisation 56B et à la forme de la chambre de désorption 56C. Il présente ainsi une forme parallélépipédique. Il délimite un volume intérieur 160 de réception des palettes 12 qui est propre à recevoir par exemple entre 2 et 50 palettes 12.

Dans l'exemple représenté sur la Figure 5, le conteneur 33 présente des parois latérales 162 ajourées délimitant des orifices de passage du gaz stérilisant. Dans cet exemple, les parois 162 sont formées par un grillage ajouré.

Le conteneur 33 comprend également une porte latérale 164 d'accès au volume intérieur 160 permettant de charger les palettes 12 dans le volume intérieur 160.

Le conteneur 33 comprend en outre des moyens 166 de préhension formés par exemple par des anneaux montés sur sa paroi supérieure.

La voie de guidage 152 est disposée au-dessus des enceintes 44, 90, 110. Elle s'étend linéairement le long de l'axe A-A' de déplacement en regard et au-dessus du poste d'entrée 20, du poste de pré-conditionnement 22, du poste de stérilisation 24, du poste de désorption 26 et du poste de sortie 28.

La longueur de la voie de guidage 152 est ainsi supérieure à quelques mètres, avantageusement à quelques dizaines de mètres.

La voie de guidage 152 comprend ainsi deux rails de guidage longitudinaux 170 portés par des poteaux verticaux 172. Les rails 170 et les poteaux 172 sont disposés de part et d'autre de l'axe A-A', transversalement par rapport aux cuves 50A, 50B, 50C.

La largeur qui sépare transversalement les rails 170 est sensiblement égale à la largeur de chaque cuve 50A, 50B, 50C. Cette largeur est supérieure à 1 m, et est par exemple comprise entre 1 m et 20m, avantageusement entre 5m et 15m.

La hauteur qui sépare les rails 170 du rebord supérieur 60 lorsque le couvercle 52A, 52B, 52C occupe sa position d'accès à la chambre 56A, 56B, 56C est sensiblement égale ou supérieure à la hauteur du conteneur 33.

La voie de guidage 152 s'étend en regard du plateau d'entrée 42 dans le poste d'entrée 20. Elle passe ensuite au-dessus des cuves de pré-conditionnement 50A, puis dans le volume confiné 140 à travers le sas amont 142 pour s'étendre successivement au-dessus des cuves de stérilisation 50B et des cuves de désorption 50C.

Elle sort ensuite du volume de confinement 140 à travers le sas aval 144 et s'étend en regard du plateau de sortie 130 du poste 28.

L'engin guidé 154 comprend un pont roulant 174 guidé par les rails 170, des moyens 176 de déplacement vertical des conteneurs 33 portés par le pont roulant 174, et des moyens 177 de propulsion du pont roulant 174 le long de la voie de guidage 152.

Le pont 174 s'étend transversalement entre les rails 170. Il est propre à rouler sur les rails 170 le long de l'axe A-A' pour passer successivement en regard des postes 20 à 28. Il est ainsi mobile exclusivement en translation le long de l'axe A-A', sa trajectoire étant asservie par la voie de guidage 152.

Le pont 174 présente une largeur sensiblement égale à la distance séparant les rails 170. Il est muni de moyens de roulage 178 sur les rails 170.

Les moyens de déplacement vertical 176 comprennent au moins un treuil 180 de traction du conteneur 33. Le treuil 180 est muni de lignes de saisie 182 propres à s'engager de manière libérable sur les moyens de préhension 166 situés sur le conteneur 33.

Lorsque les lignes de saisie 182 sont engagées sur les moyens de préhension 166, le treuil 180 est apte à porter le conteneur 33 et à la déplacer verticalement entre une position inférieure de chargement dans une enceinte 50A, 50B, 50C et une position supérieure de transport entre les postes 20 à 28, dans laquelle le conteneur 33 est déplaçable conjointement avec l'engin guidé 154 au dessus des cuves 50A, 50B, 50C.

Les moyens de propulsion 177 sont propres à déplacer le pont mobile 174 le long de la voie de guidage 152 dans deux sens opposés le long de l'axe A-A'.

Les moyens de commande 156 sont propres à activer les moyens de propulsion 177 pour piloter le déplacement de l'engin guidé 154 sur la voie de guidage 152. Ils sont propres à exécuter une séquence de prise d'un conteneur 33 dans le poste d'entrée 20, de passage du conteneur 33 contenant les palettes 12 d'objets à stériliser successivement dans le poste de pré-conditionnement 22, dans le poste de stérilisation 24, et dans le poste de désorption 26, puis une séquence de déchargement du conteneur 33 dans le poste de sortie 28.

Les moyens de commande 156 comprennent avantageusement des moyens de détrompage qui empêchent un conteneur 33 de passer dans le poste de sortie 28 s'il n'a pas subi une stérilisation dans le poste de stérilisation 24.

Un premier procédé de stérilisation d'une pluralité d'objets dans l'installation 10 va maintenant être décrit.

Initialement, les palettes 12 contenant les objets à stériliser sont déchargées du camion 43 sur le quai de déchargement 40. Elles sont ensuite transportées dans un conteneur 33 préalablement vidé et sont introduites dans le volume intérieur 160 de ce conteneur 33 en ouvrant la porte d'accès 164.

Le conteneur 33 est alors placé sur le plateau d'entrée 42 en étant positionné transversalement par rapport à l'axe A-A'.

L'engin guidé 154 est alors amené en regard et au-dessus du conteneur 33 dans le poste d'entrée 20.

A cet effet, il se déplace sous l'effet des moyens de propulsion 177 en étant guidé sur les rails 170 le long de l'axe A-A'. Lorsque le pont roulant 174 est situé au-dessus du conteneur 33, les moyens de déplacement 176 sont activés. Le treuil 180 est alors piloté pour descendre les lignes de saisie 182 et raccorder les lignes de saisie 182 aux moyens de préhension 166 du conteneur 33.

Le conteneur 33 est alors soulevé jusqu'à une position supérieure de déplacement, dans laquelle sa surface inférieure est située à une hauteur supérieure à la hauteur de la surface supérieure des couvercles 52A, 52B, 52C.

Puis, en référence à la Figure 3, les premiers vérins 74 de l'enceinte 44 de pré-conditionnement sont activés pour soulever les rails mobiles 70 et les placer en regard des rails fixes 72.

Lors de ce déplacement, les organes de roulage 66 du couvercle 52A entrent au contact des rails mobiles 70, ce qui permet de soulever le couvercle 52A à l'écart et au dessus du rebord 60.

Le deuxième vérin 76 est alors activé pour déplacer transversalement le couvercle 52A de sa position d'obturation à sa position d'accès, en faisant rouler les organes de roulage 66 sur les rails 70, 72.

Comme illustré par les Figures 2 et 4, l'ouverture supérieure 58 est alors dégagée. Le pont roulant 174 est déplacé vers l'aval le long de la voie de guidage 152 pour placer le conteneur 33 au dessus de l'ouverture supérieure 58. Puis, le treuil 180 est activé pour faire descendre le conteneur 33 vers sa position inférieure et le charger dans la chambre de pré-conditionnement 56A.

Lorsque le conteneur 33 repose sur le fond de la chambre 56A, les lignes 182 sont désengagées des moyens de préhension 166.

Ensuite, le deuxième vérin 76 est piloté pour déplacer le couvercle vers sa position d'obturation. Les rails mobiles 70 sont alors redescendus par les premiers vérins verticaux 74 pour poser le couvercle sur le rebord 60 et fermer de manière étanche la chambre 56A.

Les moyens de réglage de la température 46 et les moyens de réglage de l'hygrométrie 48 sont alors activés pour faire régner dans la chambre 56A une d'humidité relative environ comprise entre 40% et 100%, à une température comprise entre 30°C et 60°C pendant une durée supérieure à deux heures et notamment sensiblement égale à huit heures.

Lorsque le pré-conditionnement est terminé, le couvercle 52A est repassé dans sa position d'accès à la chambre 56A comme décrit précédemment. Le treuil 180 est alors à nouveau activé pour raccorder les lignes 182 sur les moyens de préhension 166 et extraire le conteneur 33 hors de la chambre 56A en le tractant verticalement vers sa position supérieure.

Le conteneur 33 est alors déplacé conjointement avec l'engin guidé 154 le long de l'axe A-A' vers le poste de stérilisation 24.

Lors de ce déplacement, le conteneur 33 pénètre dans le volume de confinement 140 à travers le sas amont 142.

Il est alors disposé en regard d'une cuve 50B de stérilisation. Comme décrit précédemment, le couvercle 52B est déplacé dans la position d'accès à la chambre de stérilisation 56B. Le conteneur 33 est alors descendu dans la chambre de stérilisation 56B et le couvercle 52B est refermé par les moyens de déplacement 54B pour obturer de manière étanche la chambre 56B.

Une fois le couvercle 52B refermé, un vide poussé est tout d'abord effectué dans la chambre de stérilisation 56B en activant la pompe à vide et en ouvrant la vanne de commande 102 de la conduite de purge 100.

Le gaz stérilisant est alors introduit à travers la conduite d'introduction 96 en ouvrant la vanne 98 pour créer une atmosphère contenant au moins 0,1% avantageusement au moins 1% de gaz stérilisant dans la chambre 56B de stérilisation, pendant une durée supérieure à deux heures et notamment sensiblement égale à huit heures.

Puis, lorsque la stérilisation est effectuée, la pompe à vide est activée et la vanne 102 de purge est ouverte pour extraire totalement le gaz contenu dans la chambre de stérilisation 56B.

La pression dans la chambre 56B est à nouveau augmentée et le couvercle 52B est ouvert. Les moyens de traction 180 soulèvent alors le conteneur 33 pour l'extraire hors de la chambre de stérilisation 56B.

Le conteneur 33 est alors déplacé par l'engin guidé 154 au-dessus des cuves 90 vers le poste de désorption 26 le long de la voie de guidage 152.

Le couvercle 52C d'une enceinte 50C de désorption est alors ouvert par les moyens de déplacement 54C, comme décrit précédemment. L'engin guidé 154 est déplacé en regard de la chambre de désorption 56C et le conteneur 33 est descendu dans la chambre 56C.

Puis, le couvercle 52C est déplacé pour fermer de manière étanche la chambre de désorption 56C.

Plusieurs injections de gaz de purge dans la chambre 56C sont réalisées par l'intermédiaire de la conduite d'injection 116, en ouvrant la vanne 118. Chaque injection est suivie d'un cycle de désorption à l'aide d'une pompe à vide en ouvrant la vanne 122 présente sur la conduite de purge 120.

Après une durée d'au moins quatre heures et notamment de trente-six heures, le couvercle 56C est ouvert et l'engin guidé 154 est placé en regard de la chambre de désorption 56C.

Le treuil 180 est activé pour soulever le conteneur 33 hors de la chambre de désorption 56C par l'intermédiaire des lignes 182.

Puis, l'engin 154 est déplacé vers l'aval sur la voie de guidage 152 et sort du volume de confinement 140 par l'intermédiaire du sas aval 144.

Lorsque l'engin guidé 154 atteint le poste de sortie, il décharge le conteneur 33, soit dans une région de stockage 132, soit dans le véhicule mobile 134, ce qui permet le transport des objets stérilisés à l'écart de l'installation 10 hors du bâtiment 18.

Le déplacement de l'engin guidé 154 entre les différents postes 20 à 28 est piloté de manière automatique par les moyens de commande 156.

L'installation 10 selon l'invention permet donc d'effectuer l'ensemble des étapes du procédé de stérilisation avec un minimum de manutention, et de manière fiable et contrôlée.

Ainsi, seules deux étapes de manutention sont nécessaires, à savoir le chargement des palettes 12 d'objets à stériliser provenant des camions 43 dans le conteneur 33, et le transport des palettes 12 d'objets stérilisés hors du bâtiment 18 à partir du poste de sortie 28.

Il est donc possible de réduire le temps et le personnel nécessaire pour mette en oeuvre la stérilisation, ce qui réduit notablement le coût, notamment lorsque les volumes d'objets à stériliser sont élevés.

De plus, la présence d'une paroi de confinement 30 délimitant un volume de confinement 140 évite l'exposition du personnel au gaz stérilisant, ce qui améliore la sécurité de l'installation 10.

Les enceintes 44, 90, 110 étant disposées côte à côte le long de l'axe A-A', l'espace occupé par l'installation 10 dans le bâtiment 18 est minimal.

La présence de moyens de commande 156 munis de moyens de détrompage assure en outre une grande fiabilité de la stérilisation puisque ces moyens évitent que des objets non stériles n'atteignent le poste de sortie 28.

Dans une variante représentée sur la Figure 6, le conteneur 33 comprend un plateau inférieur 190 de support des palettes 12 et un cadre comprenant quatre montants verticaux 192 disposés aux coins du plateau 190. Les montants verticaux 192 délimitent entre eux des ouvertures latérales totalement dégagées.

Les organes 166 de préhension sont disposés soit sur un plateau supérieur 194, soit sur des traverses raccordant les montants 192 à leurs extrémités supérieures.

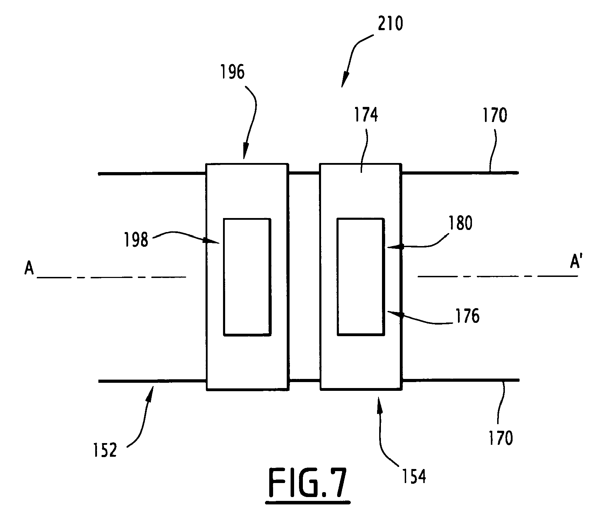

Une deuxième installation 210 selon l'invention est représentée sur la Figure 7.

A la différence de la première installation 10, les moyens de déplacement 54A, 54B, 54C de chaque couvercle 52A, 52B, 52C sont formés par un engin guidé auxiliaire 196 monté sur la voie de guidage 152 en aval de l'engin guidé 154.

L'engin guidé auxiliaire 196 est distinct de l'engin guidé 154. Comme l'engin guidé 154, il porte un treuil 198 propre à soulever chaque couvercle 52A, 52B, 52C et à le décaler longitudinalement de la cuve 50A, 50B, 50C sur laquelle il repose pour libérer l'accès à la chambre respective 56A, 56B, 56C.

Le fonctionnement de la deuxième installation 210 diffère du fonctionnement de la première installation 10 en ce que, pour passer dans la position d'accès à la chambre 56A, 56B, 56C, le couvercle 52A, 52B 52C est soulevé par le treuil 198 de l'engin guidé auxiliaire 196 et est décalé longitudinalement par rapport à l'ouverture 58, avant d'introduire le conteneur 33 dans une enceinte 44, 90, 110.

## Revendications

1. Installation (10 ; 210) de stérilisation d'une pluralité d'objets, du type comprenant :
- un poste d'entrée (20), propre à recevoir la pluralité d'objets à stériliser ;
- un poste de stérilisation (24) comprenant au moins une enceinte (90) de stérilisation destinée à recevoir la pluralité d'objets, le poste de stérilisation (24) comprenant au moins un moyen (92) d'introduction d'un fluide de stérilisation dans l'enceinte de stérilisation (90) et au moins un moyen (94) de purge du fluide de stérilisation ;
- un poste de désorption (26) du fluide de stérilisation hors de la pluralité d'objets, comprenant au moins une enceinte (110) de désorption, le poste de désorption (26) comportant au moins un moyen (114) de purge de l'enceinte de désorption ;
- un poste de sortie (28) propre à recevoir la pluralité d'objets après stérilisation ;
- des moyens (32) de transport de la pluralité d'objets, propres à déplacer successivement la pluralité d'objets depuis le poste d'entrée (20), dans le poste de stérilisation (24) et dans le poste de désorption (26), jusqu'au poste de sortie (28) ;
**caractérisée en ce que** les moyens de transport (32) comprennent :
- une voie de guidage (152) continue, le poste d'entrée (20), le poste de stérilisation (24), le poste de désorption (26) et le poste de déchargement (28) étant disposés successivement en regard de la voie de guidage (152) ; et
- au moins un engin guidé (154) par la voie de guidage (152), l'engin guidé (154) comprenant des moyens (176) de déplacement propres à déplacer la pluralité d'objets par rapport à l'engin guidé (154) entre une position de déplacement conjoint de la pluralité d'objets et de l'engin guidé (154) le long de la voie de guidage (152), et une position d'insertion de la pluralité d'objets dans chacune de l'enceinte de stérilisation (90) et de l'enceinte de désorption (110).

2. Installation (10 ; 210) selon la revendication 1, **caractérisée en ce qu'**elle comporte au moins un conteneur (33) délimitant un volume (160) de réception de la pluralité d'objets, le conteneur (33) comprenant au moins un organe (166) de raccordement libérable aux moyens de déplacement (176).

3. Installation (10 ; 210) selon l'une quelconque des revendications précédentes, **caractérisée en ce que** la voie de guidage (152) est située au-dessus de l'enceinte de stérilisation (90) et au-dessus de l'enceinte de désorption (110), l'enceinte de stérilisation (90) et l'enceinte de désorption (110) débouchant vers le haut en regard de la voie de guidage (152).

4. Installation (10 ; 210) selon la revendication 3, **caractérisée en ce que** l'engin guidé (154) comprend un pont roulant, les moyens de déplacement (176) comprenant au moins un organe de traction (182) porté par le pont roulant et propre à descendre la pluralité d'objets dans l'enceinte de stérilisation (90) et dans l'enceinte de désorption (110) et à remonter la pluralité d'objets pour les extraire hors de l'enceinte de stérilisation (90) et de l'enceinte de désorption (110).

5. Installation (10 ; 210) selon l'une quelconque des revendications précédentes, **caractérisée en ce que** l'enceinte de stérilisation (90) et l'enceinte de désorption (110) délimitent chacune une ouverture unique (58) d'accès pour l'insertion et le retrait de la pluralité d'objets.

6. Installation (10 ; 210) selon l'une quelconque des revendications précédentes, **caractérisée en ce que** la voie de guidage (152) s'étend linéairement entre le poste d'entrée (20) et le poste de sortie (28), le poste de stérilisation (24) et le poste de désorption (26) étant situés côte à côte le long de la voie de guidage (152) entre le poste d'entrée (20) et le poste de sortie (28).

7. Installation (10 ; 210) selon la revendication 6, **caractérisée en ce que** le poste de stérilisation (24) comprend une pluralité d'enceintes de stérilisation (90), le poste de désorption (26) comprenant une pluralité d'enceintes de désorption (110), les enceintes de stérilisation (90) et les enceintes de désorption (110) étant disposées successivement côte à côte de manière adjacente le long de la voie de guidage (152).

8. Installation (10 ; 210) selon l'une quelconque des revendications précédentes, **caractérisée en ce qu'**elle comprend un poste (22) de pré-conditionnement disposé en amont du poste de stérilisation (24) le long de la voie de guidage (152), le poste de pré-conditionnement (22) comprenant au moins une enceinte (44) de pré-conditionnement et des moyens (46, 48) de contrôle de l'humidité et/ou de la température dans l'enceinte de pré-conditionnement (44).

9. Installation (10 ; 210) selon l'une quelconque des revendications précédentes, **caractérisée en ce qu'**elle comprend une paroi (30) de confinement délimitant un volume de confinement (140), le poste de stérilisation (24) et le poste de désorption (26) étant situés dans le volume de confinement (140), le poste d'entrée (20) et le poste de sortie (28) étant situés hors du volume de confinement (140),
et **en ce que** la paroi de confinement (30) délimite un sas amont (142) et un sas aval (144) débouchant dans le volume de confinement (140), la voie de guidage (152) traversant successivement le sas amont (142), le volume de confinement (140) et le sas aval (144).

10. Installation (10 ; 210) selon l'une quelconque des revendications précédentes, **caractérisée en ce que** le poste de sortie (28) comprend au moins un véhicule roulant (134) délimitant une surface de chargement de la pluralité d'objets stérilisés, le véhicule roulant (134) étant mobile entre une position de réception de la pluralité d'objets dans laquelle la surface de chargement s'étend en regard de la voie de guidage (152) pour être chargée par les moyens de transport (32) et une position à l'écart de la voie de guidage (152).

11. Installation (10 ; 210) selon l'une quelconque des revendications précédentes, **caractérisée en ce que** l'enceinte de stérilisation (90) délimite une chambre (56B) de stérilisation propre à recevoir la pluralité d'objets et comprend un ouvrant (52B) d'obturation étanche de la chambre de stérilisation (56B), le poste de stérilisation (24) comprenant des moyens (54B) de déplacement de l'ouvrant (52B) propre à déplacer l'ouvrant entre une position d'accès à la chambre de stérilisation (56B) et une position d'obturation de la chambre de stérilisation (56B).

12. Installation (10 ; 210) selon la revendication 11, **caractérisée en ce que**, dans la position d'accès, la chambre de stérilisation (56B) débouche en regard de la voie de guidage (152), l'ouvrant (52B) étant décalé transversalement par rapport à la voie de guidage (152).

13. Installation (210) selon la revendication 11 ou 12, **caractérisée en ce que** les moyens de déplacement de l'ouvrant (52B) comprennent un engin (196) auxiliaire guidé par la voie de guidage.

14. Procédé de stérilisation d'une pluralité d'objets dans une installation (10 ; 210) selon l'une quelconque des revendications précédentes, **caractérisé en ce qu'**il comprend les étapes suivantes :
- saisie de la pluralité d'objets dans le poste d'entrée (20) par les moyens de déplacement (176) portés par l'engin guidé (154),
- déplacement de l'engin guidé (154) sur la voie de guidage (152) pour le placer en regard du poste de stérilisation (24) ;
- introduction de la pluralité d'objets dans l'enceinte de stérilisation (90) par les moyens de déplacement (176) portés par l'engin guidé (154) ;
- injection de fluide stérilisant dans l'enceinte de stérilisation (90) pour stériliser la pluralité d'objets dans l'enceinte de stérilisation (90) ;
- extraction de la pluralité d'objets hors de l'enceinte de stérilisation (90) par les moyens de déplacement (176) portés par l'engin guidé (154) ;
- déplacement de l'engin guidé (154) sur la voie de guidage (152) pour le placer en regard du poste de désorption (26) ;
- introduction de la pluralité d'objets dans l'enceinte de désorption (110) par les moyens de déplacement (176) portés par l'engin guidé (154) ;
- désorption du fluide de stérilisation hors de la pluralité d'objets dans l'enceinte de désorption (110) ;
- extraction de la pluralité d'objets hors de l'enceinte de désorption (110) par les moyens de déplacement (176) portés par l'engin guidé (154) ;
- déplacement de l'engin guidé (154) sur la voie de guidage (152) jusqu'au poste de sortie (28) ;
- dépôt de la pluralité d'objets stérilisés dans le poste de sortie (28) par les moyens de déplacement (176) portés par l'engin guidé (154).

## Claims

1. Installation (10; 210) for sterilising a plurality of objects, of the type comprising:
- an input station (20) which is capable of receiving the plurality of objects to be sterilised;
- a sterilisation station (24) which comprises at least one sterilisation compound (90) which is intended to receive the plurality of objects, the sterilisation station (24) comprising at least one means (92) for introducing a sterilising fluid into the sterilisation compound (90) and at least one means (94) for discharging the sterilising fluid;
- a desorption station (26) for desorbing the sterilising fluid from the plurality of objects, comprising at least one desorption compound (110), the desorption station (26) comprising at least one means (114) for draining the desorption compound;
- an output station (28) which is capable of receiving the plurality of objects after sterilisation;
- means (32) which are for transporting the plurality of objects and which are capable of successively moving the plurality of objects from the input station (20) into the sterilisation station (24) and into the desorption station (26) as far as the output station (28);
**characterised in that** the transport means (32) comprise:
- a continuous guiding path (152), the input station (20), sterilisation station (24), desorption station (26) and unloading station (28) being arranged successively opposite the guiding path (152); and
- at least one device (154) which is guided by the guiding path (152), the guided device (154) comprising movement means (176) which are capable of moving the plurality of objects relative to the guided device (154) between a position for common movement of the plurality of objects and the guided device (154) along the guiding path (152) and a position for insertion of the plurality of objects in each of the sterilisation compound (90) and the desorption compound (110).

2. Installation (10; 210) according to claim 1, **characterised in that** it comprises at least one container (33) which delimits a volume (160) for receiving the plurality of objects, the container (33) comprising at least one member (166) for releasable connection to the movement means (176).

3. Installation (10; 210) according to either of the preceding claims, **characterised in that** the guiding path (152) is located above the sterilisation compound (90) and above the desorption chamber (110), the sterilisation compound (90) and the desorption compound (110) opening upwards opposite the guiding path (152).

4. Installation (10; 210) according to claim 3, **characterised in that** the guided device (154) comprises a travelling platform, the movement means (176) comprising at least one traction member (182) which is carried by the travelling platform and which is capable of lowering the plurality of objects into the sterilisation compound (90) and into the desorption compound (110), and of raising the plurality of objects in order to remove them from the sterilisation compound (90) and the desorption compound (110).

5. Installation (10; 210) according to any one of the preceding claims, **characterised in that** the sterilisation compound (90) and the desorption compound (110) each delimit a single access opening (58) for inserting and removing the plurality of objects.

6. Installation (10; 210) according to any one of the preceding claims, **characterised in that** the guiding path (152) extends linearly between the input station (20) and the output station (28), the sterilisation station (24) and the desorption station (26) being located side by side along the guiding path (152) between the input station (20) and the output station (28).

7. Installation (10; 210) according to claim 6, **characterised in that** the sterilisation station (24) comprises a plurality of sterilisation compounds (90), the desorption station (26) comprising a plurality of desorption compounds (110), the sterilisation compounds (90) and the desorption compounds (110) being arranged successively side by side in an adjacent manner along the guiding path (152).

8. Installation (10; 210) according to any one of the preceding claims, **characterised in that** it comprises a pre-processing station (22) which is arranged upstream of the sterilisation station (24) along the guiding path (152), the pre-processing station (22) comprising at least one pre-processing compound (44) and means (46, 48) for controlling the humidity and/or the temperature in the pre-processing chamber (44).

9. Installation (10; 210) according to any one of the preceding claims, **characterised in that** it comprises a confinement wall (30) which delimits a confinement volume (140), the sterilisation station (24) and the desorption station (26) being located in the confinement volume (140), the input station (20) and the output station (28) being located outside the confinement volume (140),
and **in that** the confinement wall (30) delimits an upstream lock chamber (142) and a downstream lock chamber (144) opening in the confinement volume (140), the guiding path (152) extending successively through the upstream lock chamber (142), the confinement volume (140) and the downstream lock chamber (144).

10. Installation (10; 210) according to any one of the preceding claims, **characterised in that** the output station (28) comprises at least one moving vehicle (134) which delimits a surface for loading the plurality of sterilised objects, the moving vehicle (134) being movable between a position for receiving the plurality of objects, in which the loading surface extends opposite the guiding path (152) in order to be loaded by the transport means (32), and a position remote from the guiding path (152).

11. Installation (10; 210) according to any one of the preceding claims, **characterised in that** the sterilisation compound (90) delimits a sterilisation chamber (56B) which is capable of receiving the plurality of objects and comprises a lid (52B) for sealingly closing the sterilisation chamber (56B), the sterilisation station (24) comprising means (54B) for moving the lid (52B) which are capable of moving the lid between a position for access to the sterilisation chamber (56B) and a position for closing the sterilisation chamber (56B).

12. Installation (10; 210) according to claim 11, **characterised in that**, in the access position, the sterilisation chamber (56B) opens opposite the guiding path (152), the lid (52B) being transversely displaced relative to the guiding path (152).

13. Installation (210) according to claim 11 or claim 12, **characterised in that** the means for moving the lid (52B) comprise an auxiliary device (196) which is guided by the guiding path.

14. Method for sterilising a plurality of objects in an installation (10; 210) according to any one of the preceding claims, **characterised in that** it comprises the following steps:
- the plurality of objects being taken from the input station (20) by the movement means (176) carried by the guided device (154);
- moving the guided device (154) over the guiding path (152) in order to place it opposite the sterilisation station (24);
- the plurality of objects being introduced into the sterilisation compound (90) by the movement means (176) carried by the guided device (154);
- injecting sterilising fluid into the sterilisation compound (90) in order to sterilise the plurality of objects in the sterilisation compound (90);
- the plurality of objects being removed from the sterilisation compound (90) by the movement means (176) carried by the guided device (154);
- moving the guided device (154) over the guiding path (152) in order to place it opposite the desorption station (26);
- the plurality of objects being introduced into the desorption compound (110) by the movement means (176) carried by the guided device (154);
- desorption of the sterilising fluid from the plurality of objects in the desorption compound (110);
- the plurality of objects being removed from the desorption compound (110) by the movement means (176) carried by the guided device (154);
- moving the guided device (154) over the guiding path (152) as far as the output station (28);
- the plurality of sterilised objects being deposited in the output station (28) by the movement means (176) carried by the guided device (154).

## Patentansprüche

1. Anlage (10; 210) zum Sterilisieren einer Mehrzahl von Gegenständen, vom Typ enthaltend:
- eine Einlassstation (20), die dazu geeignet ist, die Mehrzahl von zu sterilisierenden Gegenständen aufzunehmen;
- eine Sterilisationsstation (24) mit zumindest einem Sterilisationsraum (90), der dazu bestimmt ist, die Mehrzahl von Gegenständen aufzunehmen, wobei die Sterilisationsstation (24) zumindest ein Mittel (92) zum Einführen eines Sterilisationsfluids in den Sterilisationsraum (90) und zumindest ein Mittel (94) zum Ablassen des Sterilisationsfluids aufweist;
- eine Desorptionsstation (26) zur Desorption des Sterilisationsfluids aus der Mehrzahl von Gegenständen, enthaltend zumindest einen Desorptionsraum (110), wobei die Desorptionsstation (26) zumindest ein Mittel (114) zum Entleeren des Desorptionsraums aufweist;
- eine Auslassstation (28), die dazu geeignet ist, die Mehrzahl von Gegenständen nach der Sterilisation aufzunehmen;
- Mittel (32) zum Transportieren der Mehrzahl von Gegenständen, die dazu geeignet sind, die Mehrzahl von Gegenständen nacheinander von der Einlassstation (20) in die Sterilisationsstation (24) und in die Desorptionsstation (26) bis hin zur Auslassstation (28) zu verlagern;
**dadurch gekennzeichnet, dass**
die Transportmittel (32) aufweisen:
- eine durchgehende Führungsbahn (152), wobei die Einlassstation (20), die Sterilisationsstation (24), die Desorptionsstation (26) und die Entladestation (28) der Führungsbahn (152) gegenüberliegend nacheinander angeordnet sind; und
- zumindest eine über die Führungsbahn (152) geführte Leiteinrichtung (154), wobei die Leiteinrichtung (154) Verlagerungsmittel (176) aufweist, die dazu geeignet sind, die Mehrzahl von Gegenständen bezüglich der Leiteinrichtung (154) zwischen einer Stellung zur gemeinsamen Verlagerung der Mehrzahl von Gegenständen und der Leiteinrichtung (154) entlang der Führungsbahn (152) und einer Stellung zum Einführen der Mehrzahl von Gegenständen jeweils in den Sterilisationsraum (90) und in den Desorptionsraum (110) zu verlagern.

2. Anlage (10; 210) nach Anspruch 1,
**dadurch gekennzeichnet, dass**
sie zumindest einen Container (33) aufweist, der ein Fassungsvolumen (160) zum Aufnehmen der Mehrzahl von Gegenständen eingrenzt, wobei der Container (33) zumindest ein Organ (166) zum lösbaren Verbinden mit den Verlagerungsmitteln (176) aufweist.

3. Anlage (10; 210) nach einem der vorangehenden Ansprüche,
**dadurch gekennzeichnet, dass**
die Führungsbahn (152) sich über dem Sterilisationsraum (90) und über dem Desorptionsraum (110) befindet, wobei der Sterilisationsraum (90) und der Desorptionsraum (110) gegenüber der Führungsbahn (152) nach oben ausmünden.

4. Anlage (10; 210) nach Anspruch 3,
**dadurch gekennzeichnet, dass**
die Leiteinrichtung (154) einen Laufkran aufweist, wobei die Verlagerungsmittel (176) zumindest ein Zugglied (182) aufweisen, das von dem Laufkran getragen wird und dazu geeignet ist, die Mehrzahl von Gegenständen in den Sterilisationsraum (90) und in den Desorptionsraum (110) abzusenken und die Mehrzahl von Gegenständen anzuheben, um sie aus dem Sterilisationsraum (90) und aus dem Desorptionsraum (110) herauszuführen.

5. Anlage (10; 210) nach einem der vorangehenden Ansprüche,
**dadurch gekennzeichnet, dass**
der Sterilisationsraum (90) und der Desorptionsraum (110) jeweils eine einzige Zugangsöffnung (58) zum Einführen und Herausführen der Mehrzahl von Gegenständen eingrenzen.

6. Anlage (10; 210) nach einem der vorangehenden Ansprüche,
**dadurch gekennzeichnet, dass**
die Führungsbahn (152) linear zwischen der Einlassstation (20) und der Auslassstation (28) verläuft, wobei die Sterilisationsstation (24) und die Desorptionsstation (26) entlang der Führungsbahn (152) zwischen der Einlassstation (20) und der Auslassstation (28) nebeneinander liegen.

7. Anlage (10; 210) nach Anspruch 6,
**dadurch gekennzeichnet, dass**
die Sterilisationsstation (24) eine Mehrzahl von Sterilisationsräumen (90) aufweist, wobei die Desorptionsstation (26) eine Mehrzahl von Desorptionsräumen (110) aufweist, wobei die Sterilisationsräume (90) und die Desorptionsräume (110) nacheinander so nebeneinanderliegend angeordnet sind, dass sie entlang der Führungsbahn (152) aneinandergrenzen.

8. Anlage (10; 210) nach einem der vorangehenden Ansprüche,
**dadurch gekennzeichnet, dass**
sie eine Vorbehandlungsstation (22) aufweist, die der Sterilisationsstation (24) entlang der Führungsbahn (152) vorgelagert ist, wobei die Vorbehandlungsstation (22) zumindest einen Vorbehandlungsraum (44) und Mittel (46, 48) zur Kontrolle der Feuchtigkeit und/oder der Temperatur in dem Vorbehandlungsraum (44) aufweist.

9. Anlage (10; 210) nach einem der vorangehenden Ansprüche,
**dadurch gekennzeichnet, dass**
sie eine Sicherheitswand (30) aufweist, die einen Sicherheitsraum (140) eingrenzt, wobei die Sterilisationsstation (24) und die Desorptionsstation (26) in dem Sicherheitsraum (140) liegen und die Einlassstation (20) und die Auslassstation (28) außerhalb des Sicherheitsraumes (140) liegen,
und dass von der Sicherheitswand (30) eine vorgelagerte Schleuse (142) und eine nachgelagerte Schleuse (144) eingegrenzt sind, die in den Sicherheitsraum (140) münden, wobei die Führungsbahn (152) sich nacheinander durch die vorgelagerte Schleuse (142), den Sicherheitsraum (140) und die nachgelagerte Schleuse (144) erstreckt.

10. Anlage (10; 210) nach einem der vorangehenden Ansprüche,
**dadurch gekennzeichnet, dass**
die Auslassstation (28) zumindest einen Rollwagen (134) aufweist, der eine Ladefläche für die Mehrzahl von sterilisierten Gegenständen eingrenzt, wobei der Rollwagen (134) zwischen einer Stellung zum Aufnehmen der Mehrzahl von Gegenständen, in welcher die Ladefläche der Führungsbahn (152) gegenüberliegt, um über die Transportmittel (32) beladen zu werden, und einer von der Führungsbahn (152) entfernt liegenden Stellung beweglich ist.

11. Anlage (10; 210) nach einem der vorangehenden Ansprüche,
**dadurch gekennzeichnet, dass**
der Sterilisationsraum (90) eine Sterilisationskammer (56B) eingrenzt, die dazu geeignet ist, die Mehrzahl von Gegenständen aufzunehmen, und ein Schließteil (52B) zum dichten Verschließen der Sterilisationskammer (56B) aufweist, wobei die Sterilisationsstation (24) Mittel (54B) zum Verlagern des Schließteils (52B) aufweist, die dazu geeignet sind, das Schließteil zwischen einer Zugangsstellung zur Sterilisationskammer (56B) und einer Stellung zum Verschlie-βen der Sterilisationskammer (56B) zu verlagern.

12. Anlage (10; 210) nach Anspruch 11,
**dadurch gekennzeichnet, dass**
in der Zugangsstellung die Sterilisationskammer (56B) gegenüber der Führungsbahn (152) ausmündet, wobei das Schließteil (52B) quer zur Führungsbahn (152) verlagert wird.

13. Anlage (210) nach Anspruch 11 oder 12,
**dadurch gekennzeichnet, dass**
die Mittel zum Verlagern des Schließteils (52B) eine Hilfsleiteinrichtung (196) aufweisen, die von der Führungsbahn geführt wird.

14. Verfahren zum Sterilisieren einer Mehrzahl von Gegenständen in
einer Anlage (10; 210) nach einem der vorangehenden Ansprüche, **dadurch gekennzeichnet, dass**
es die nachfolgenden Schritte umfasst:
- Ergreifen der Mehrzahl von Gegenständen in der Einlassstation (20) über die Verlagerungsmittel (176), die von der Leiteinrichtung (154) getragen werden,
- Verlagern der Leiteinrichtung (154) auf der Führungsbahn (152), um sie der Sterilisationsstation (24) gegenüberliegend anzuordnen;
- Einführen der Mehrzahl von Gegenständen in den Sterilisationsraum (90) über die Verlagerungsmittel (176), die von der Leiteinrichtung (154) getragen werden;
- Einspritzen von Sterilisationsfluid in den Sterilisationsraum (90) zum Sterilisieren der Mehrzahl von Gegenständen im Sterilisationsraum (90);
- Herausnehmen der Mehrzahl von Gegenständen aus dem Sterilisationsraum (90) mittels der von der Leiteinrichtung (154) getragenen Verlagerungsmittel (176);
- Verlagern der Leiteinrichtung (154) auf der Führungsbahn (152), um sie der Desorptionsstation (26) gegenüberliegend anzuordnen;
- Einführen der Mehrzahl von Gegenständen in den Desorptionsraum (110) über die Verlagerungsmittel (176), die von der Leiteinrichtung (154) getragen werden;
- Desorption des Sterilisationsfluids aus der Mehrzahl von Gegenständen im Desorptionsraum (110);
- Herausnehmen der Mehrzahl von Gegenständen aus dem Desorptionsraum (110) mittels der von der Leiteinrichtung (154) getragenen Verlagerungsmittel (176);
- Verlagern der Leiteinrichtung (154) auf der Führungsbahn (152) bis hin zur Auslassstation (28);
- Ablegen der Mehrzahl von sterilisierten Gegenständen in der Auslassstation (28) über die von der Leiteinrichtung (154) getragenen Verlagerungsmittel (176).
